# EUROPEAN PATENT APPLICATION

(11) **EP 1 359 417 A2**
(43) Date of publication of application: **05.11.2003**
(21) Application number: 03252720.2
(22) Date of filing: 30.04.2003
(51) Int. Cl.: G01N 33/52, G01N 33/543

(54) **Devices and methods for analyte concentration determination**

(30) Priority: 01.05.2002 US 137146
(71) Applicant: Lifescan, Inc., Milpitas, California 95035-6312 (US)
(72) Inventor: Eyster, Curt R., San Jose, California 95139 (US); Wallace, Brian, San Jose, California 95118 (US)
(74) Representative: Mercer, Christopher Paul

(57) **Abstract**

Devices, systems and methods for the determination of the concentration of at least one analyte in a physiological sample are provided. The subject devices include a matrix having at least one calibration mark and at least one testing area having reagent compositions for determining the concentration of an analyte in the sample. The subject systems include a subject device and a meter configured to determine the concentration of at least one analyte in a sample applied to the device. The subject methods include (1) providing a subject device, (2) associating the device with a meter, (3) illuminating at least one calibration mark with light, (4) detecting light from at least one calibration mark, and (5) calibrating the meter based on the detected at least one calibration mark. Also provided are kits for use in practicing the subject methods. Also provided are kits for use in practicing the subject methods.

## Description

### FIELD OF THE INVENTION

The field of this invention is analyte concentration determination.

### BACKGROUND OF THE INVENTION

Analyte concentration determination in physiological samples is of ever increasing importance to today's society. Such assays find use in a variety of application settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like.

In response to this growing importance of analyte concentration determination, a variety of analyte concentration determination protocols and devices for both clinical and home testing have been developed. Of great interest and use in this area are optical based analyte determination devices and methods in which a sample is illuminated with light and reflected light therefrom is detected, where the amount of detected light is related to analyte concentration. Of increasing interest in such optical based measurement protocols is the use of assay systems that employ analyte concentration measurement devices configured as test strips or cards, and which are read automatically by a suitable analyte concentration determination device, i.e., a meter. Typically, a physiological sample such as blood, blood derivatives, interstitial fluid, urine, etc., is introduced to a test strip, where the sample reacts with certain reagents or components associated with the testing area of the test strip to produce a color reaction. Analyte concentration is measured automatically by associating the test strip with a meter that is essentially a reflectance photometer and which determines analyte concentration by irradiating the testing area of the test strip, detecting reflected light therefrom and relating the amount of reflected light to analyte concentration.

Whether the test is performed in the home, physician's office, clinic or hospital, accuracy and reproducibility of the determined analyte concentration are extremely important, especially for individuals suffering from life-threatening illnesses who are dependent upon the results of these analyte concentration determinations for illness management, for example, diabetics where the concentration of glucose determines insulin intake amounts, etc. However, the test strips used in these tests, by their nature, do not lend themselves to large-scale manufacture with adequate test strip-to-test strip reproducibility from one batch to the next. Consequently, it is necessary to assign to each lot of test strips a calibration code that corrects for this variability by calibrating the meter used to read the test strip according to this calibration code. The calibration code may be positioned in any convenient location, such as the instructions that accompany such test strips. Usually, the user manually enters the code into the meter when he or she begins a new test. If the user fails to enter a new calibration code or enters an incorrect one, the resulting Value of analyte concentration will be incorrect.

Attempts to provide automatic meter calibration that does not involve the user manually inputting the calibration code have been made. However, while effective, such attempts suffer from disadvantages.

U.S. Pat. No. 4,476,149, to Poppe et al., discloses an analysis test strip and process for making it that includes on-strip calibration information. The strip includes a "test field" in which the analysis takes place and a batch-specific bar code, which provides calibration information specific to strips made in a particular batch. (See also U.S. Pat. Nos. 4,510,383 and 4,592,893.) In principle, the process provides a strip whose calibration is "transparent" to the user; i.e., the user is unaware of the calibration step. While that is a highly desirable result, it comes at a high price. The bar code must be printed very precisely, with tight tolerances on the width and spacing of the bars, over the entire length of the web that constitutes a single batch of (uncut) strips. Moreover, the printing must be done in a way that does not change the characteristics of the test field. Furthermore, the meter must have a sophisticated optical system in order to read the tightly-spaced bar code reliably.

U.S. Pat. No. 5,281,395, to Markart et al., discusses the practical problems raised by the strip of Poppe, et al. and addresses some of them with a two-strip system. The "test carrier" contains the reagent for reacting with the analyte to be measured and the "code carrier" has the calibration bar code that is characteristic of a particular batch. Each carrier also has a machine-readable batch identification. This approach reduces the technical difficulties and expense involved in manufacturing the strips of Poppe et al; however, it requires the use of a second strip in order to calibrate the meter.

Connolly, in PCT Application W0 96/13707, published on May 9, 1996, discloses an apparatus and method for detecting various analytes in body fluids, using dry test strips. In one embodiment, test strips are color coded to identify the test that a particular strip is intended for. Thus, a blue strip may measure glucose and a red strip cholesterol. The colors are divided into shades, for example 64 shades of blue represent 64 different lot numbers of glucose strips. The apparatus has a memory module which stores a lot number. If the lot number measured from the strip doesn't match the lot number in the memory module, the test isn't performed. This approach requires that each batch of test strips have a memory module, which is inserted into the apparatus before the strips of that batch can be used.

Still further, U.S. Patent No. 5,989, 917 to McAleer et al. discloses a meter that reads a calibration code from a test strip container, where the calibration code is in the form of a bar code, magnetic stripe, memory chip or resonant wire loop. However, each of these formats suffers from disadvantages. For example, as described above, a bar code must be printed very precisely, with tight tolerances on the width and spacing of the bars. Moreover, the meter must have a sophisticated optical system having moveable parts to scan across the bar code in order to read the tightly-spaced bar code reliably. Accordingly, such a system increases manufacturing costs.

As such, there is continued interest in the development of new devices and methods for analyte concentration determination that provide easy calibration of an analyte concentration determination device, i.e., an analyte concentration determination meter. Of particular interest would be the development of such devices and methods that do not place excessive demands on the manufacturing process of either the meter or the test strip, that enables the use and detection of a wide variety of calibration marks from a matrix of a test strip before calculating analyte concentration thereby eliminating the manual inputting of a calibration code by the user who may be unaware or forgetful that calibration is needed, and which may determined the concentration of more than analyte.

### SUMMARY OF THE INVENTION

Devices, systems and methods for use in the determination of the concentration of at least one analyte in a physiological sample are provided. The subject devices include a matrix having at least one calibration mark and at least one testing area, wherein the at least one testing area has reagent compositions for determining the concentration of an analyte in the physiological sample. The subject systems include a subject device and a meter configured to determine the concentration of at least one analyte in the physiological sample applied to the device.

The subject invention also includes methods for calibrating a meter. The subject methods include (1) providing a subject device, (2) associating the device with a meter, (3) illuminating the at least one calibration mark with light, (4) detecting light from the at least one calibration mark, and (5) calibrating the meter based on the detected calibration mark(s). The methods also include contacting the testing area of the matrix with physiological sample and providing a calibrated concentration determination of at least one analyte in the physiological sample. Also provided are kits for use in practicing the subject methods.

### BRIEF DESCRIPTION OF THE DRAWINGS

Figure 1 shows an exemplary embodiment of a device according to the subject invention configured as a test strip.
Figure 2 shows an enlarged plan view of an exemplary embodiment of the matrix of the device of Figure 1.
Figure 3 shows another enlarged plan view of an exemplary embodiment of the matrix of the device of Figure 1.
Figure 4 shows another enlarged plan view of an exemplary embodiment of the matrix of the device of Figure 1.
Figure 5 shows another enlarged plan view of an exemplary embodiment of the matrix of the device of Figure 1.
Figure 6 shows a schematic illustration of an exemplary embodiment of a meter according to the subject invention having a portion of a test strip associated therewith.
Figure 7A-7E shows enlarged plan views of exemplary embodiments of various configurations of the detector array of the meter of Figure 6.
Figure 8 shows an enlarged plan view of an exemplary embodiment of the matrix of the device of Figure I having multiple calibration marks and multiple testing areas.
Figure 9 shows an enlarged plan view of another exemplary embodiment of the matrix of the device of Figure 1 having two different calibration marks, each in duplicate and multiple testing areas.
Figure 10 shows an enlarged plan view of another exemplary embodiment of the matrix of the device of Figure 1 having three different calibration marks.
Figure 11 shows an enlarged plan view of another exemplary embodiment of the matrix of the device of Figure 1 having a calibration mark in the pattern of a number.

### DETAILED DESCRIPTION OF THE INVENTION

Devices, systems and methods for use in the determination of the concentration of at least one analyte in a physiological sample are provided. The subject devices include a matrix having at least one calibration mark and at least one testing area, wherein the at least one testing area has reagent compositions for determining the concentration of an analyte in the physiological sample. The subject systems include a subject device and a meter configured to determine the concentration of at least one analyte in the physiological sample applied to the device.

The subject invention also includes methods for calibrating a meter. The subject methods include (1) providing a subject device, (2) associating the device with a meter, (3) illuminating the at least one calibration mark with light, (4) detecting light from the at least one calibration mark, and (5) calibrating the meter based on the detected calibration mark(s). The methods also include contacting the testing area of the matrix with physiological sample and providing a calibrated concentration determination of at least one analyte in the physiological sample. Also provided are kits for use in practicing the subject methods.

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a reagent" includes a plurality of such reagents and reference to "the device" includes reference to one or more devices and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEVICES

As summarized above, devices used in the concentration determination of an analyte in a physiological sample are provided. Generally, the subject devices include a matrix having at least one calibration mark that is used to calibrate a meter, and at least one testing area having reagent compositions for analyte concentration determination. Accordingly, one or more components, features or aspects of a meter may be calibrated according to the at least one calibration mark so that the meter may provide at least one calibrated analyte concentration. As mentioned above, the at least one testing area of the matrix includes reagent compositions for analyte concentration determination. In those embodiments having more than one testing area, the reagent compositions present in any of the testing areas may be the same or different reagent composition from that present in any other testing area. For example, in certain embodiments at least two testing areas have the same reagent compositions in different concentrations, or may have different reagent compositions for determining the concentration of different analytes, or all may be the same, as will be described in greater detail below. In further describing the subject invention, the subject devices will be described first in greater detail, followed by systems that include the subject devices and meters used with the subject devices. Next, a description of methods for calibrating a meter according to the subject invention, and kits for use in practicing the subject methods, are described.

### Analyte Concentration Measurement Devices

The subject invention includes analyte concentration measurement devices and more particularly photometric or colorimetric (used herein interchangeably) analyte concentration measurement devices. The devices employed in the subject invention are generally made up of at least the following components: (1) a matrix 11 having at least one calibration mark positioned thereon and at least one testing area having reagent compositions (not shown as a structural component) that typically include one or more members of an analyte oxidation signal producing system, and (2) a support element 12, to which the matrix of the device is associated. The devices are configured and adapted to be received in a meter, as described below, for automatically determining the concentration of an analyte. In further describing the subject analyte concentration measurement devices, reference to analyte concentration measurement devices configured as test strips will be used for exemplary purposes only and is in no way intended to limit the scope of the invention.

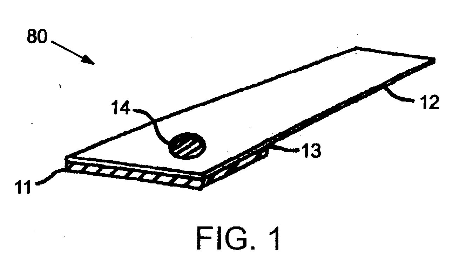

An exemplary embodiment of a subject test strip is shown in Figure 1. Figure 1 shows test strip 80 in which matrix 11 is positioned at one end of support element 12 with adhesive 13. A hole 14 is present in support element 12 in the area of matrix 11 in which a sample can be applied to one side of matrix 11 and a reaction can be detected therefrom. Usually, sample is applied to one side of matrix 11 and a reaction is detected at another or opposite side of matrix 11, however, other configurations and methods are possible as well. The components of test strip 80 will now be described in more detail.

### Matrix

Matrix 11 is made of an inert material which provides a support for at least one calibration mark and various members of the signal producing system, described below, as well as the light absorbing or chromogenic product, i.e., the indicator, produced by the signal producing system. Matrix 11 is configured to provide a location for at least one calibration mark and a location for the physiological sample, *e.g.,* blood, application, as well as the detection of the light-absorbing product produced by the indicator of the signal producing system. As such, the latter location may be characterized as the testing, detection or measurement area of the test strip (used herein interchangeably). As such, matrix 11 is one that is permissive of aqueous fluid flow through it and provides sufficient void space for the chemical reactions of the signal producing system to take place. A number of different matrices have been developed for use in various analyte determination assays, which matrices may differ in terms of materials, dimensions and the like, where matrices suitable for use in the subject invention include, but are not limited to, those described in U.S. Patent Nos.: 4,734,360; 4,900,666; 4,935,346; 5,059,394; 5,304,468; 5,306,623; 5,418,142; 5,426,032; 5,515,170; 5,526,120; 5,563,042; 5,620,863; 5,573,452; 5,780,304; 5,789,255; 5,843,691; 5,846,486; 5,968,836 and 5,972,294; the disclosures of which are herein incorporated by reference.

In principle, the nature of matrix 11 is not critical to the subject test strips and therefore is chosen with respect to other factors, including the nature of the instrument which is used to read the test strip, convenience and the like. As such, the dimensions and porosity of the matrix may vary greatly, where matrix 11 may or may not have pores and/or a porosity gradient, *e.g*. with larger pores near or at the sample application region and smaller pores at the detection region. The materials from which matrix 11 may be fabricated vary, and include polymers, *e.g*. polysulfone, polyamides, cellulose or absorbent paper, and the like, where the material may or may not be functionalized to provide for covalent or non-covalent attachment of the various members of the signal producing system.

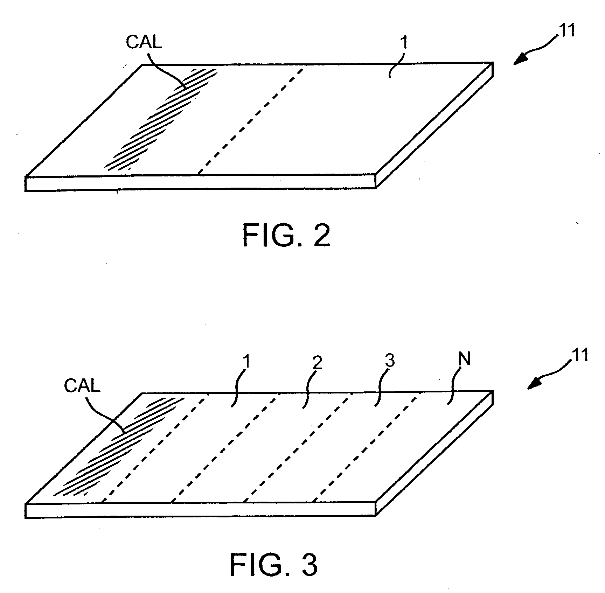

As mentioned above, matrix 11 includes at least one calibration mark CAL (represented here as a hatched mark) and at least one testing area 1, as shown in the enlarged view of an exemplary embodiment of matrix 11 in Figure 2. The number of calibration marks and testing areas may vary according to the particular application of the test strip, where the number of calibration marks and testing areas present on matrix 11 may range from about 1 to thousands of calibration marks or more and from about 1 to thousands of testing areas or more. Figures 3, 4 and 5 and Figures 8 through 11 show additional exemplary embodiments of matrix 11 having at least one calibration mark CAL and at least one testing area configured in various manners.

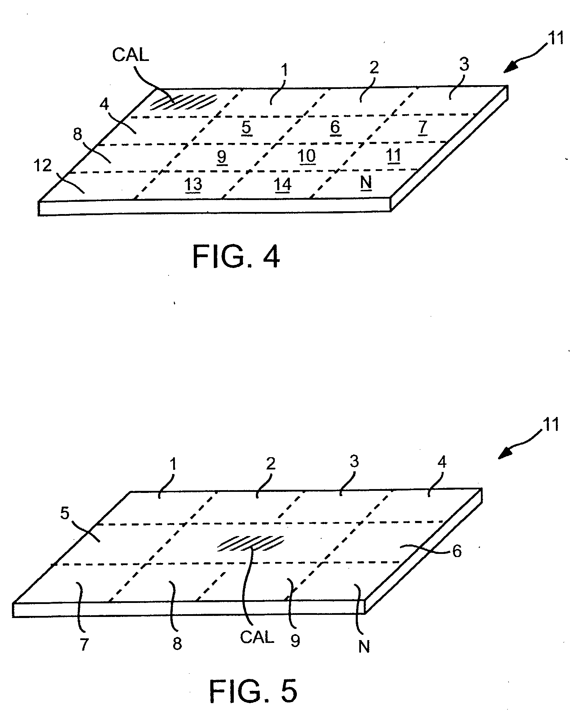

More specifically, Figure 3 shows matrix 11 having a calibration mark CAL and testing areas 1-N arranged in parallel on matrix 11. In all embodiments, one or more of the testing areas may be the same or one or more may be different. Figure 4 shows matrix 11 having a calibration mark CAL and testing areas 1-N arranged in a grid-like arrangement on matrix 11. Figure 5 shows matrix 11 having calibration mark CAL positioned substantially in the center of matrix 11 and testing areas 1-N positioned around calibration mark CAL.

A feature of the subject methods is that the use of a plurality of detectors, as will be described in greater detail below, enables the detection of a plurality of calibration marks positioned on a matrix, where in many embodiments at least one calibration mark(s) form a pattern such as a number or letter or the like on the matrix, where such can be detected using a plurality of detectors instead of a single detector as sued in prior art devices. Accordingly, matrix 11 may include more than one calibration mark, where the marks present may be the same, e.g., for quality control purposes, or different, e.g., the ratio of two or more of the marks may indicate particular calibration parameters or each calibration mark may relate to a particular testing area such as a particular analyte to be tested. In certain embodiments, the detection of a particular calibration mark indicates what analyte a particular testing area is assaying for, in other words indicates to the meter to use a particular algorithm or computation related to a particular testing area/analyte of interest.

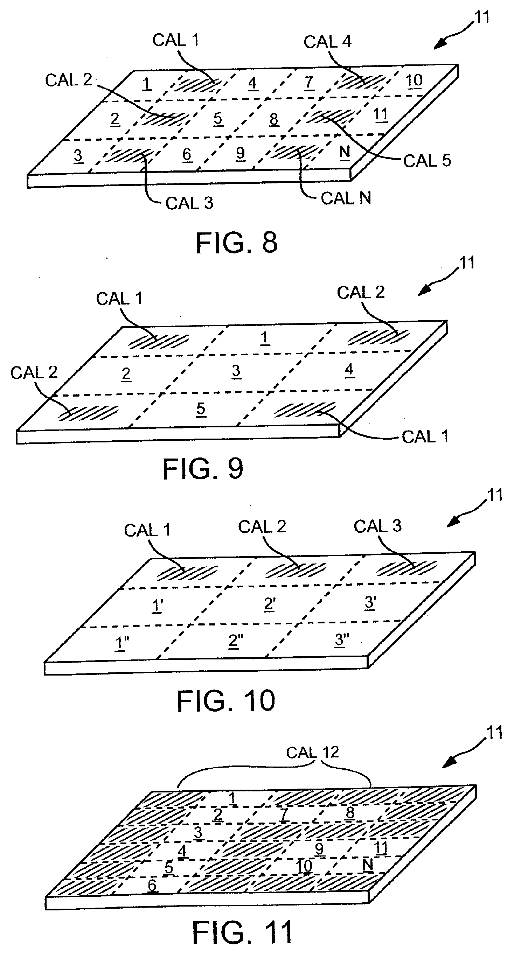

Figure 8 shows matrix 11 having calibration marks CAL 1-CAL N and testing areas 1-N. As mentioned above, some or all of at least one calibration marks may be the same or may some or all may be different. For example, a calibration mark may be particular to the testing area to which it is adjacent. For example, in certain embodiments, a testing area may assay for an inhibiting substance such as acetaminophen (which inhibits glucose) and an adjacent testing area may assay for glucose. As such, if acetaminophen is detected, for example in a particular amount, at least one calibration mark associated therewith provides a correction factor to be used in the computation or algorithm used for the analyte concentration determination of glucose.

Figure 9 shows yet another configuration of matrix 11 having two calibration marks CAL 1 and CAL 2 and five testing areas. In use, for example, calibration parameters may be indicated by the ratio of the two marks, where duplicates are provided for quality control purposes and/or the positioning of one or more of the marks may indicate what analyte is being assayed for in a particular testing area. For example, as shown in Figure 10, calibration mark CAL 1 may indicate to the meter that testing areas 1' and 1" are related to CAL 1 and/or assay for a particular analyte, calibration mark CAL2 may indicate to the meter that testing areas 2' and 2" are related to CAL 2 and/or assay for a particular analyte and calibration mark CAL 3 may indicate to the meter that testing areas 3' and 3" are related to CAL 3 and/or assay for a particular analyte.

Figure 11 shows an exemplary embodiment of matrix 11 having a calibration mark CAL 12 in the pattern of a number, shown here as number 12 and testing areas 1-N. Of course, any pattern of number(s), letter(s) or combination(s) thereof may be used to indicate a calibration mark or a plurality of calibration marks on a matrix.

It will be apparent that a variety of different arrangements of one or more calibration marks and one or more testing areas are possible, where the above described embodiments are exemplary only and in no way intended to limit the scope of the invention.

The at least one calibration mark is one that is readable by a photometric detector, and therefore may be correctly characterized as a photometrically or optically readable calibration mark. Accordingly, the at least one calibration mark is one that indicates distinct, respective calibration information related to the particular test strip carrying the at least one calibration mark, i.e., meter parameters, used to calibrate a meter.

The at least one photometrically readable calibration mark may be made distinctive using any convenient technique. For example, the at least one photometrically readable calibration mark may be distinctive based on size, shape, the number of marks, the wavelength of detectable light therefrom, hue, shading, a pattern of one mark or a pattern made of a plurality of marks, the ratio of two or more marks, the position of one or more marks, a gradation of color, a gradation of hue or a gradation of shading, etc., and any combination thereof. In certain embodiments, the mark becomes readable by the application of sample thereto, e.g., at least one calibration mark is activated by the interaction with fluid or sample, e.g., is bleached or faded or made visible, etc.

The at least one testing area of the matrix includes reagent compositions or testing reagents, as described above, for analyte concentration determination. The one or more testing areas are usually segregated, though not always, from the area or areas of the matrix having a calibration mark, and/or from each other, to prevent cross-contamination from area to area. Such segregation may be accomplished in any convenient manner. For example, each area may be defined by a chemical and/or physical barrier such as a hydrophobic barrier, crimping of the matrix, or the like (see for example European patent application No. 02258366.0 and U.S. Patent No. 5,843,691, the disclosures of which are herein incorporated by reference).

Where more than one testing area is present, each testing area may be the same or different with respect to the reagent compositions, i.e., at least two of the testing areas may differ. In certain embodiments of the invention, the reagent composition are the same in all the disparate testing areas of matrix 11, e.g., in multi-use test strips. In other embodiments, e.g., where the test strip is employed to simultaneously assay for a panel or plurality of different analytes, the reagent composition will differ among some or all disparate testing areas. In other words, at least two different reagent compositions will be present in different testing areas of the test strip, where the number of different reagent compositions may be as great as the number of different testing areas of the test strip. In certain embodiments, the strip may simultaneously assay for one or more substances that may interfere with the one or more analyte of interest and/or may be used to determine the hematocrit level in a sample applied to the test strip.

In certain embodiments, one or more testing areas may include an inhibiting component that retards the reaction between some or all of the components of the reagent compositions. Accordingly, in certain other embodiments, the reagent composition may be the same in all or substantially all the testing areas (i.e., the testing areas assay for the same analyte), but the composition in adjoining testing areas may increase or decrease stepwise, in inhibitor concentration. As described, in the testing area, the testing reagents react with the analyte of interest, e.g., glucose, to produce a detectable product. In this particular instance, detectable product is produced if analyte, e.g., glucose, concentration is large enough to overcome the inhibitor level in that particular testing area. Thus, each succeeding testing area, if made of increasing amounts of inhibitor, requires a greater glucose concentration in the sample to cause a detectable product (see for example, U.S. Patent No. 5,843,691, the disclosure of which is herein incorporated by reference).

In many embodiments, one or more of the testing areas may be a control area such that it has compositions of known analyte concentration, i.e., a positive control area, or has no reagent compositions, i.e., a negative control area.

Accordingly, in the embodiment shown in Figure 3, matrix 11 has a calibration mark CAL and the testing areas 1-N may have different reagent compositions for assaying for different analytes. For example, first testing area 1 may have a reagent composition for the concentration determination of a first analyte, such as glucose, second testing area 2 may have a reagent composition for the concentration determination of a second analyte such as ketones, third testing area 3 may have a reagent composition for the concentration determination of a third analyte such as cholesterol, and an Nth testing area n may have a reagent composition for the concentration determination of an Nth analyte such as a substance that is known to interfere with the determination of the concentration of one or more of the analytes assayed in one or more of the testing areas, e.g., may be known to interfere with the concentration determination of one or more of glucose, ketones and cholesterol. Alternatively, one or more of the testing areas, e.g., testing area 1, may have the same reagent composition as one or more of any of the other testing areas, e.g., testing area 2, where the reagent compositions may differ in concentration of one or more components. As mentioned above, one or more of the testing areas may be a control area having known analyte concentration, i.e., a positive control area, or has no reagent compositions, i.e., a negative control area.

As described above, the reagent compositions present in the at least one testing area include one or more members of a signal producing system which produces a detectable product in response to the presence of a target analyte, which detectable product can be used to derive the amount of analyte present in the assayed sample. In the subject test strips, the one or more members of the signal producing system are associated with, *e.g.*, covalently or non-covalently attached to, at least a portion of matrix 11 (*i.e*., the detection, testing or measurement area), and in certain embodiments associated with substantially all of matrix 11.

In certain embodiments, e.g., where glucose is the analyte of interest, the signal producing system is an analyte oxidation signal producing system. By analyte oxidation signal producing system is meant that in generating the detectable signal from which the analyte concentration in the sample is derived, the analyte is oxidized by one or more suitable enzymes to produce an oxidized form of the analyte and a corresponding or proportional amount of hydrogen peroxide. The hydrogen peroxide is then employed, in turn, to generate the detectable product from one or more indicator compounds, where the amount of detectable product generated by the signal measuring system, *i.e*. the signal, is then related to the amount of analyte in the initial sample. As such, the analyte oxidation signal producing systems present in the test strips are also correctly characterized as hydrogen peroxide based signal producing systems.

As indicated above, the hydrogen peroxide based signal producing systems include a first enzyme that oxidizes the analyte and produces a corresponding amount of hydrogen peroxide, i.e., the amount of hydrogen peroxide that is produced is proportional to the amount of analyte present in the sample. The specific nature of this first enzyme necessarily depends on the nature of the analyte being assayed but is generally an oxidase. As such, the first enzyme may be: glucose oxidase (where the analyte is glucose); cholesterol oxidase (where the analyte is cholesterol); alcohol oxidase (where the analyte is alcohol); lactate oxidase (where the analyte is lactate) and the like. Other oxidizing enzymes for use with these and other analytes of interest are known to those of skill in the art and may also be employed. In those preferred embodiments where the reagent test strip is designed for the detection of glucose concentration, the first enzyme is glucose oxidase. The glucose oxidase may be obtained from any convenient source, *e.g.* a naturally occurring source such as Aspergillus niger or Penicillum, or recombinantly produced.

A second enzyme of the signal producing system may be an enzyme that catalyzes the conversion of one or more indicator compounds into a detectable product in the presence of hydrogen peroxide, where the amount of detectable product that is produced by this reaction is proportional to the amount of hydrogen peroxide that is present. This second enzyme is generally a peroxidase, where suitable peroxidases include: horseradish peroxidase (HRP), soy peroxidase, recombinantly produced peroxidase and synthetic analogs having peroxidative activity and the like. See *e.g.,* Y. Ci, F. Wang; Analytica Chimica Acta, 233 (1990), 299-302.

The indicator compound or compounds, *e.g.,* substrates, are ones that are either formed or decomposed by the hydrogen peroxide in the presence of the peroxidase to produce an indicator dye that absorbs light in a predetermined wavelength range. Preferably the indicator dye absorbs strongly at a wavelength different from that at which the sample or the testing reagent absorbs strongly. The oxidized form of the indicator may be a colored, faintly-colored, or colorless final product that evidences a change in color of the testing side of the membrane. That is to say, the testing reagent can indicate the presence of glucose in a sample by a colored area being bleached or, alternatively, by a colorless area developing color.

Indicator compounds that are useful in the present invention include both one-and two-component chromogenic substrates. One-component systems include aromatic amines, aromatic alcohols, azines, and benzidines, such as tetramethyl benzidine-HCI. Suitable two-component systems include those in which one component is MBTH, an MBTH derivative (see for example those disclosed in EP-A-0 781 350 08/302,575, incorporated herein by reference), or 4-aminoantipyrine and the other component is an aromatic amine, aromatic alcohol, conjugated amine, conjugated alcohol or aromatic or aliphatic aldehyde. Exemplary two-component systems are 3-methyl-2-benzothiazolinone hydrazone hydrochloride (MBTH) combined with 3-dimethylaminobenzoic acid (DMAB); MBTH combined with 3,5-dichloro-2-hydroxybenzene-sulfonic acid (DCHBS); and 3-methyl-2-benzothiazolinonehydrazone N-sulfonyl benzenesulfonate monosodium (MBTHSB) combined with 8-anilino-1 naphthalene sulfonic acid ammonium (ANS). In certain embodiments, the dye couple MBTHSB-ANS is preferred.

In yet other embodiments, signal producing systems that produce a fluorescent detectable product (or detectable non- fluorescent substance, *e.g.* in a fluorescent background) may be employed, such as those described in: Kiyoshi Zaitsu, Yosuke Ohkura: New fluorogenic substrates for Horseradish Peroxidase: rapid and sensitive assay for hydrogen peroxide and the Peroxidase. Analytical Biochemistry (1980) 109, 109-113.

In using such a colorimetric test strip, sample is allowed to react with the members of the signal producing system to produce a detectable product that is present in an amount proportional to the initial amount present in the sample. The amount of detectable product, *i.e*., signal produced by the signal producing system, is then determined and related to the amount of analyte in the initial sample. In many embodiments, sample is applied to one side or a first side of matrix 11 and the amount of detectable product is then determined at another or second side of matrix 11, where in many embodiments the amount of detectable product is determined on a side opposite the first side. In certain embodiments, automated meters that perform the above mentioned detection and relation steps are employed, as noted above and which will be described in greater detail below. The above described reaction, detection and relating steps, are further described in U.S. Patent Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference.

### Support Element

As mentioned above, matrix 11 is usually attached to a support element 12. Support element 12 may be of a material that is sufficiently rigid to be inserted into an automated device such as a meter without undue bending or kinking. Matrix 11 may be attached to support element 12 by any convenient mechanisms, e.g., clamps, adhesive, etc., herein shown attached using an adhesive 13. In many embodiments, support member 12 is made of material such as polyolefins, *e.g*., polyethylene or polypropylene, polystyrene or polyesters. Consequently, the length of the support element 12 typically dictates or corresponds to the length of the test strip. In the example shown in Figure 1, one support element 12 is employed on one side of matrix 11. However, in certain embodiments, another support element is attached to the other side of matrix 11 so as to "sandwich" the matrix between two support elements.

As described above, support element 12 is usually configured to enable test strip 80 to be used with a meter. As such, support element 12, and thus test strip 80, may be in the form of a substantially rectangular or square-like strip, where the dimensions of support element 12 vary according to a variety of factors, as will be apparent to those of skill in the art.

The size of test strip 80 may vary according to a variety of factors such as the dimensions of the meter with which it is used, the number of testing areas of the test strip, etc. Typically, the length of the test strip 80 ranges from about 5 mm to about 80 mm, usually from about 15 mm to about 65 mm, the width of test strip 80 typically ranges from about 5 mm to about 20 mm, usually from about 6 mm to about 12 mm and the thickness of test strip 80 typically ranges from about 0.1 mm to about 0.8 mm, usually from about 0.2 mm to about 0.4 mm.

In such a test strip having the dimensions described above, the length of the matrix typically ranges from about 2.0 mm to about 30.0 mm, usually from about 5.0 mm to about 10.0 mm, the width of the matrix typically ranges from about 2.0 mm to about 30.0 mm, usually from about 10.0 mm to about 20.0 mm and the thickness of the matrix typically ranges from about 0.1 mm to about 1.0 mm, usually from about 0.2 mm to about 0.4 mm.

### SYSTEMS

As summarized above, the subject invention provides systems that include the subject devices, where such systems at least include a subject device, e.g., configured as a test strip as described above, and an analyte concentration determination meter, i.e., an optical meter, configured for use with a subject device for determining the concentration of at least one analyte in a physiological sample applied to the analyte concentration measurement device.

The optical meters of the subject invention include at least one light source for illuminating both the at least one calibration mark and the at least one testing area of the matrix of a subject device that is associated or mated with the meter, a detector array made-up of at least two detectors, at least one detector configured to detect light from at least one calibration mark and at least another detector configured to detect reflected light from the at least one testing area of an analyte concentration measurement device, where one or more of the detectors of the array may be configured to detect light from both the at least one calibration mark and the at least one testing area. Typically, such a detector array includes a plurality of detectors configured to detect one or more calibration marks, where the use of a plurality of detectors enables the indication of calibration parameters based on the detection of a particular mark or plurality of marks made distinctive by shape, pattern, positioning, gradation of color, hue, shading etc., such as calibration mark shown in Figure 11 in a pattern of a number, where such would not be detectable using a single detector as will be apparent to those of skill in the art. The subject meters also include means for calibrating at least one component, aspect or feature of the meter based on the detected at least one calibration mark and means for determining a calibrated concentration of at least one analyte in the physiological sample applied to the device.

The size of the subject meters will vary depending on a variety of factors such as the size of the test strips used with the meters, the shape and dimensions of the devices used, etc. However generally, the meters of the subject invention are small enough to be portable or easily moveable. By way of example, the length of a subject meter typically ranges from about 50 mm to about 150 mm and more usually from about 60 mm to about 100 mm, the width typically ranges from about 40 mm to about 100 mm and more usually from about 60 mm to about 90 mm and the thickness or diameter typically ranges from about 10 mm to about 30 mm and more usually from about 15 mm to about 25 mm.

Likewise, the shape of the subject meters will vary, where the shape may range from simple to complex. In many embodiments, the subject meters will assume a circular, oblong, oval, square or rectangular shape, although other shapes are possible as well, such as irregular or complex shapes.

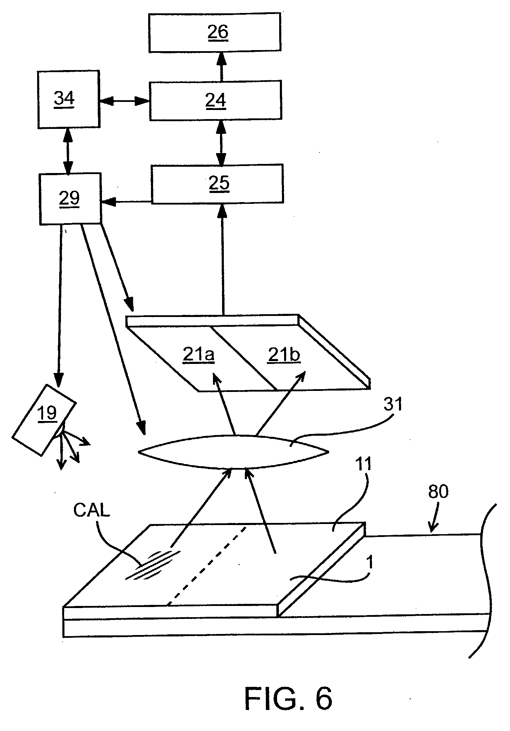

The subject meters will now be further described with reference to the Figures, where like numerals represent like components or features. Figure 6 shows meter 20 schematically illustrated. A partial view of test strip 80 is shown operatively associated with meter 20. In this particular embodiment matrix 11 of test strip 80 has an area having a calibration mark CAL and an area that is a testing area 1.

As mentioned above, meter 20 includes at least one light source 19. At least one light source 19 projects light onto the area of the test strip, e.g., matrix 11, having at least one calibration mark and the testing area, where the same or different light source may project light onto the one or more testing area of the matrix at the same or different time as the projection of light onto at least one calibration mark. At least one light source 19 typically includes a light emitting diode (LED) or any other convenient light source such as a laser diode, a phototransistor, and the like. Usually, the light source contains two or more LED sources or the like, e.g., in certain embodiments light source 19 has three or more LED sources or the like, or light source 19 may be a single diode capable of emitting two or more distinct wavelengths of light. The at least one light source is usually capable of emitting light at wavelengths ranging from about 400 nm to about 1000 nm, usually from about 500 nm to about 940 nm. For example, where two distinct wavelengths are employed, the light source is capable of emitting light at about 635 nm and about 700 nm and in many embodiments the light source is capable of emitting light of wavelengths at about 660 nm and 940 nm, where in certain embodiments the light source is capable of emitting light at wavelengths at about 525 nm, 630 nm and 940 nm. It will be apparent that the wavelengths described herein are for exemplary purposes only and are in no way intended to limit the scope of the invention as many other combinations of wavelengths are possible as well. Commercially available light sources that produce wavelengths of light described above include, but are not limited to, those provided by OSRAM Sylvania, Inc., LEDtronics, Inc., Agilent Technologies, Inc., and Stanley Electric Sales of America.

The subject meters also include a detector array 21 made-up of at least two detectors: at least one detector or a first detector 21a for detecting light from at least one calibration mark on the matrix of the test strip and at least another detector or a second detector 21b for detecting light from at least one testing area of the test strip, where one or more of the detectors that make-up the detector array may be capable of detecting light from at least one calibration mark and at least one testing area. The number of detectors that make-up the detector array will vary according to the configuration of the matrix, the number and configuration of the at least one calibration mark, etc., but usually will be at least two detectors.

Accordingly, a feature of the subject invention is that at least one detector, typically a plurality of detectors, of the detector array is capable of detecting light, e.g., diffusely reflected light, from at least one calibration mark positioned on a matrix of a test strip, where such light is reflected due to the light source irradiating the photometrically readable calibration mark. In this regard, the at least one detector that detects light corresponding to a photometrically readable calibration mark on a matrix may also be correctly characterized as a calibration detector. Using a plurality of detectors advantageously enables the detection of a pattern, such as in the form of a number or letter or the like, or gradation of color, shading, hue, etc, as described, such that such a calibration mark may be determined based on the reflected light detected from each area. The one or more remaining detectors detect light from the one or more testing areas of the matrix, respectively, and therefore may also be correctly characterized as testing detectors. However, one or more detectors may detect light from both a calibration mark and a testing area.

Accordingly, the number of detectors of a detector array employed in the subject invention will vary depending on a variety of factors such as the size and shape of the matrix, the number of calibration marks and testing areas thereon, etc. and will usually be equal to or greater than the number of testing areas such that each detector detects light from at least one testing area, and in many embodiments at least one other calibration detector for detecting light from at least one calibration mark, where one or more testing detectors may also serve as calibration detectors, and vice versa.

In certain embodiments, about three detectors or more are present, e.g., in a linear or triangular arrangement. In many embodiments, about four detectors or more are present (e.g., configured in a 2X2 arrangement), where the number of detectors may range from about 2 detectors to about 100 or more detectors, where the number of detectors employed will vary depending on the size and shape of the testing area, etc. In other words, the number of individual detectors that make-up the detector array is related to the number of discrete sections or testing areas of the matrix and the number of calibration marks of the matrix, where testing and calibration areas may overlap in some instances. In certain embodiments of the subject invention employing a charge coupled device ("CCD") camera array, the array may have about 1,000 or more detectors such that in certain embodiments thousands of detectors may be present, e.g., arranged in a 512 x 494 arrangement or 1024 x 2048 arrangement.

The configuration of the detectors that make up the detector array may vary according to a variety of factors such as the size and shape of the calibration area and testing area(s) of the matrix, the position of at least one calibration mark(s) on the matrix, and the like, however the detector array is configured as a single unit made of at least two detectors with one detector of the array configured to detect a photometrically readable calibration mark from the matrix. That is, the individual detectors are associated together to form one piece or one component, e.g., in a matrix or grid-like arrangement or pattern.

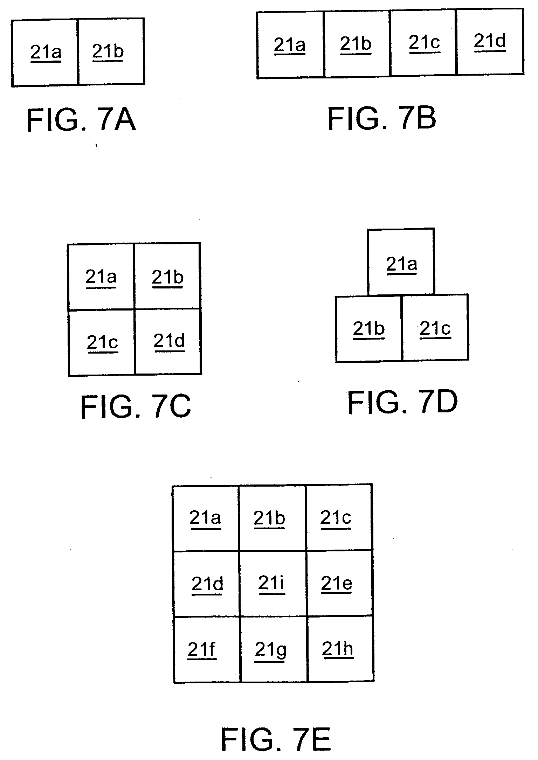

Figures 7A-7E show plan views of exemplary embodiments of the subject detector array in a variety of configurations, where such configurations are exemplary only and are in no way intended to limit the scope of the invention. Accordingly, Figure 7A shows two detectors, detector 21a and detector 21b configured in a 2 x 2 arrangement, where at least one of the detectors is configured to detect a photometrically readable calibration mark from the matrix of a test strip and at least one detector is configured to detect light from a testing area of the test strip.

Figure 7B shows another embodiment having four detectors, detector 21a, detector 21b, detector 21c and detector21d, configured in a linear arrangement, where at least one of the detectors is configured to detect a photometrically readable calibration mark from the matrix of a test strip and at least one detector is configured to detect light from a testing area of the test strip.

Figure 7C shows another embodiment having four detectors, detector 21 a, detector 21b, detector 21c and detector21d, configured in a matrix-type arrangement, where at least one of the detectors is configured to detect a photometrically readable calibration mark from the matrix of a test strip and at least one detector is configured to detect light from a testing area of the test strip.

Figure 7D shows another embodiment having three detectors, calibration detector 21a, detector 21b and detector 21c, configured in a triangular or non-linear arrangement, where at least one of the detectors is configured to detect a photometrically readable calibration mark from the matrix of a test strip and at least one detector is configured to detect light from a testing area of the test strip.

Figure 7E shows yet another embodiment of array detector 21 having nine detectors, detectors 21a- 21i, configured in a matrix or grid-type arrangement, where at least one of the detectors is configured to detect a photometrically readable calibration mark from the matrix of a test strip and at least one detector is configured to detect light from a testing area of the test strip.

As is apparent, the number of individual detectors and the configuration thereof employed to make up a subject detector array may vary as appropriate. Each detector of detector array 21 is capable of detecting or intercepting light, e.g., diffusely reflected light, such that the detectors are photodetectors. (It will be apparent that such detectors may also be configured to detect transmitted light.)

As described above, at least one calibration mark on the matrix is a distinct mark or plurality of marks, where each distinct mark(s) indicates distinct, respective parameters relating to the meter. As such, the calibration detector is one that has suitable resolution to adequately detect the photometrically readable calibration mark so that corresponding calibration information may be indicated therefrom to calibrate a meter.

The subject meters also may include imaging optics 31 or one or more light pipes or the like for imaging reflected light from specific areas of the matrix onto specific, respective detectors. Accordingly, as shown in Figure 6, imaging optics 31 is configured to image light from at least one calibration mark CAL positioned on matrix 11 onto at least one calibration detector and image light from at least one testing area onto at least one detector. Optional imaging optics 31 may take the form of one or more lenses or mirrors or light pipes or combination thereof. In certain embodiments, a different imaging optics may be employed to image reflected light from the one or more testing areas onto one or more appropriate detectors than the imaging optics used to image light onto one or more appropriate calibration detectors.

The subject meters also include means for calibrating a meter based on the particular calibration mark detected by the calibration detector. This means is generally a digital integrated circuit 29, where such calibration means 29 is under the control of a software program and thus is suitably programmed to execute all of the steps or functions required of it to receive a signal from the calibration detector, relate the received signal to particular calibration information or set of parameters, and carry out all the steps necessary to provide a calibrated analyte concentration measurement based on at least one calibration mark. In other words, calibration means 29 is configured to implement or follow an algorithm stored in the meter for calibrating the meter according to a detected calibration mark. More specifically, at least one calibration mark indicates particular parameters to which the meter is calibrated to provide an accurate analyte concentration determination for the particular test strip. For example, the calibration information corresponding to a particular calibration mark may require the meter to calibrate, adjust or modify one or more components, aspects or features of the meter or to use a particular calibration value or variable in the analyte concentration determination computation, where such calibration information is tailored to the particular test strip used with the meter. Calibration means 29 usually reads the output of a signal conversion element such as analog/digital converter 25 which converts an analog signal from the calibration detector 21Cal of the detector array 21 to a digital signal. Accordingly, calibration means 29 is capable of carrying out all the steps necessary to provide a calibrated analyte concentration measurement based on a detected calibration mark specific to the test strip used with the meter.

Calibration means 29 is thus capable of calibrating the meter in a number of ways, depending on at least one calibration mark and corresponding meter parameters, i.e., depending on the particular requirements of the test strip to be used with the meter. That is, calibration means 29 is capable of calibrating or adjusting one or more components, aspects or features, etc., of the meter to provide an accurate, i.e., calibrated, analyte concentration determination. For example, calibration means 29 is capable of calibrating one or more of the following: (1) the at least one light source 19, e.g., the intensity of light, the duration of light, depth of the light, etc., (2) the detector array 21, e.g., gain, offset (3) the imaging optics 31, e.g., positioning, focus etc., (4) the means for determining analyte concentration(s), e.g., the algorithm used to compute analyte concentration, etc., and the like. By calibrating an algorithm is meant any adjustment, change or modification to an algorithm including, but not limited to, selecting an appropriate algorithm, modifying an algorithm, incorporating a variable or value such as a correction value or the like into an algorithm, etc., or any such adjustment or selection of an algorithm as is necessary to provide a calibrated analyte concentration determination, i.e., an analyte concentration determination that is more accurate than one determined without calibration. For example, a particular calibration mark may indicate a hematocrit correction factor or value to be incorporated into an algorithm for a particular level hematocrit detected in a sample or a particular calibration mark may indicate an interfering substance correction factor or value to be incorporated into an algorithm for a particular level of interfering substance detected in a sample.

In addition to the above described means for calibrating a meter based on the particular calibration mark detected by the calibration detector, the subject meters also include means for determining the concentration of at least one analyte in a sample based on the reflected light detected from the one or more testing area(s) of the matrix of a test strip. This means is generally a digital integrated circuit 24, where such an integrated circuit 24 is under the control of a software program and thus is suitably programmed to execute all of the steps or functions required of it, or any hardware or software combination that will perform such required functions. In other words, analyte concentration determination means 24 is configured to carry-out or follow an algorithm stored in the meter for determining the concentration of at least one analyte. Analyte concentration determination means 24 is shown in figure 10 as a separate component from calibration means 29, but in certain embodiments means for calibration and means for determining the concentration of an analyte may be the same integrated circuit. Accordingly, analyte concentration determination means 24 is capable of carrying out all the steps necessary to determine a calibrated analyte concentration measurement.

The subject meters also include program and data memory 34, which may be a digital integrated circuit, that stores data and the digital integrated circuit(s) operating program(s). For example, program and data memory 34 may store calibration information, i.e., meter parameters, correction values, algorithms, etc., relating to particular calibration marks, operating programs, etc.

Reporting means 26 is configured to communicate the results of the analyte concentration measurement determination, error messages, etc., to the user and may take various hard copy and soft copy forms. Usually it is a visual display such as a liquid crystal display (LCD) or light emitting diode (LED) display, but it may also be a tape printer, audible signal, or the like.

### METHODS

The subject invention also provides methods for calibrating an analyte concentration determination meter. Specifically, the subject invention provides methods for calibrating a meter according to at least one calibration mark positioned on a matrix of a subject device and determining a calibrated concentration measurement value of at least one analyte in a physiological sample applied to the device.

Generally, a subject device, e.g., usually configured as a test strip, and a subject meter are provided. The at least one calibration mark positioned on the matrix of the test strip is detected, the detected calibration mark is related to particular calibration information or a particular set of meter parameters or a correction factor or value, etc., and the meter is calibration based on such calibration information. The calibration of the meter may be performed before, after or both before and after sample is applied to the test strip, for example the sample itself may provide calibration information or the like. For example, in certain embodiments, sample is applied to the test strip and the test strip may then be operatively inserted into the meter, where at least one calibration mark is illuminated and detected and the meter calibrated accordingly. In other embodiments, the test strip is operatively inserted into the meter and sample is then applied, where at least one calibration mark may be detected before or after sample introduction, or both before and after sample introduction, to the matrix. The subject methods will be further described with respect to sample application to the test strip before the test strip is inserted into the meter for the sake of brevity and is in no way intended to limit the scope of the invention.

Sample is introduced to the test strip and more specifically to the matrix of the test strip, where typically sample is confined to each area, i.e., the at least one calibration area and the one or more testing areas, by chemical and/or physical barriers to prevent cross- contamination. Specifically, physiological sample is applied to the matrix such that sample reacts with the members of the signal producing system of the matrix to produce a detectable product that is present in an amount proportional to the initial amount present in the sample, as described above. The amount of sample that is introduced may vary, but generally ranges from about 0.1 to 25 µl, usually from about 5 to 10 µl. The sample may be introduced to the matrix using any convenient protocol, where the sample may be injected, allowed to wick, or otherwise introduced. In certain embodiments, the sample may be introduced and cause a bleaching or fading of a calibration mark, where such bleaching or degree of such may indicate particular calibration parameters or may cause a calibration mark to develop.

Following introduction of the sample to the matrix, the meter is calibrated according to at least one calibration mark positioned on the matrix such that at least one calibration mark positioned on the matrix of the test strip is detected and related to particular calibration information, where such information is used to calibrate the meter. In certain embodiments, as described above, at least one calibration mark is made detectable by the application of sample thereto.

Accordingly, at least one detector, i.e., at least one calibration detector, of the detector array of the meter detects the at least one photometrically readable calibration mark from the matrix of the device, where the at least one calibration mark provides information for calibrating one or more components, aspects or features of the meter. As such, light illuminates the at least one calibration mark and the light reflected (or absorbed) therefrom is detected by one or more calibration detectors of the detector array of the meter. Light may illuminate the entire matrix at one time, or may illuminate only the area of at least one calibration mark(s) first, such that the at least one testing area of the meter may be illuminated at a time thereafter, with the same or different light source with light of the same or different wavelength(s).

The wavelength(s) of light used to illuminate the at least one calibration mark may be the same or different from the wavelength(s) used to illuminate the one or more testing area(s) of the matrix. Light of any suitable wavelength(s) may be used to illuminate the at least one calibration mark (where certain wavelengths may be used to illuminate certain calibration marks and other wavelengths used to illuminate other calibration marks), where such wavelength(s) is dependent upon the type of calibration mark, the type of detector, etc., where wavelength(s) of light ranging from about 400 nm to about 1000 nm are typically used to illuminate at least one calibration mark. In certain embodiments, light of more than one wavelength is used to illuminate at least one calibration mark, e.g., a first wavelength ranging from about 400 nm to about 600 nm and a second wavelength ranging from about 700 nm to about 940 nm.

Light from the at least one calibration mark is then detected by at least one calibration detector to provide a detected calibration signal that is related to particular calibration information or parameters stored by the meter for calibrating the meter according to the parameters designated by the at least one calibration mark. As such, the meter is calibrated or adjusted according to these parameters. That is, one or more components, aspects or features of the meter is calibrated or adjusted based on the at least one calibration mark identified as corresponding to the particular test strip. For example, one or more of the following may be calibrated according to at least one calibration mark: (1) the light source, e.g., the intensity of light, the duration of light, depth, etc., (2) one or more detectors of the detector array, e.g., gain, offset (3) the imaging optics, e.g., positioning, focus, etc., (4) the integrated circuit(s), e.g., the algorithm used to compute analyte concentration, etc., and the like. As described above, calibrating an algorithm is meant any adjustment, change or modification to an algorithm including, but not limited to, selecting an appropriate algorithm, modifying an algorithm, incorporating a variable or value such as a correction value into an algorithm, etc., or any such adjustment or selection of an algorithm as is necessary to provide a calibrated analyte concentration determination, i.e., an analyte concentration determination that is more accurate than one determined without calibration. For example, in one embodiment, meter calibration includes determining or identifying, based upon a calibration mark, an appropriate variable or value such as a correction value, e.g., for hematocrit correction or interfering substance correction or the like, that is used in an analyte concentration determination algorithm or calculation employed by the meter to compute analyte concentration.

After the meter has been calibrated, e.g., after the light source is modified and/or the detectors adjusted and/or a specific algorithm or variable has been determined, etc., light from the at least one testing area is detected by at least one detector of the detector array and at least one analyte concentration is determined, i.e., at least one calibrated analyte concentration determination is made, where the at least one analyte concentration is determined based upon the reflected light detected from the at least one testing area of the matrix. In certain embodiments, light may be detected from the testing area(s) before or at substantially the same time as light is detected from the calibration mark, where the detected light may be used in an algorithm or computation after the meter has been calibrated based on the calibration mark.

Accordingly, light illuminates the testing area(s) of the matrix, where usually light at wavelength(s) ranging from about 400 nm to about 1000 nm, usually from about 500 nm to about 940 nm illuminates the testing area(s), where more than one wavelength may be employed. For example, where two distinct wavelengths are employed for the testing area(s), light at about 635 nm and about 700 nm is employed and in many embodiments light of wavelengths at about 660 nm and 940 nm are employed, where in certain embodiments light of wavelengths at about 525 nm, 630 nm and 940 nm is employed. It will be apparent that the wavelengths described herein are for exemplary purposes only and are in no way intended to limit the scope of the invention as many other combinations of wavelengths are possible as well. In certain embodiments, light may illuminate testing areas at different times and different wavelengths may be used to illuminate different testing areas.

Light is then detected from the at least one testing area, where light from each of the testing areas, if more than one, is detected. The detected light from the at least one testing area is related to the amount of analyte in the sample. In certain embodiments, the analyte concentration of a plurality of analytes is determined such that the matrix includes a plurality of testing areas, where at least two of which may have different reagent compositions for determining the analyte concentration of at least two or more analytes. In certain embodiments, one or more of the testing areas is a control area such as a positive and/or negative control. In such instances, light is detected therefrom, where the meter may then determine whether the test is in error or not, where a determination of error is reported to the user.

The subject methods may also include imaging light from specific areas of the test strip onto the specific detector(s) of the detector array. For example, imaging optics may be used to image light from at least one calibration mark onto the calibration detector and light from each testing area onto each respective detector.

The subject methods may also include determining whether a sufficient amount of sample has been applied to the matrix as described in copending European patent application claiming priority from USSN 10/137,598 [Attorney's ref: P033788EP].

It will be apparent to those of skill in the art that the above described steps for calibrating a meter and analyte concentration determination may be altered or modified, e.g., the order thereof may be modified. For example, certain steps described herein as according serially may occur substantially simultaneously, and the like.

### KITS

Finally, kits for practicing the subject methods are provided. The subject kits include at least one device, e.g., configured as a test strip, of the subject invention, where the subject kits typically include a plurality of subject devices. The subject kits may also include a subject meter. The subject kits may further include an element for obtaining a physiological sample. For example, where the physiological sample is blood, the subject kits may further include an element for obtaining a blood sample, such as a lance for sticking a finger, a lance actuation means, and the like. In addition, the subject kits may include a control solution or standard, e.g., a control solution that has a known analyte concentration such as a known glucose concentration. The kits may further include instructions for using the at least one device for calibrating a meter and determining the presence and/or concentration of at least one analyte in a physiological sample applied to the device. The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

It is evident from the above description and discussion that the above described invention provides devices and methods for easily calibrating an analyte concentration determination device. The above described invention provides a number of advantages, including, but not limited to, a test strip that integrates the calibration function and the testing function on the matrix, ease of use, ease and low cost of manufacture, automation of the calibration process and the ability to provide a calibrated analyte concentration determination for at least one analyte. The subject invention also enables a wide variety of photometrically readable calibration marks to be employed on the matrix of the test strip, where such can be easily detected by a detector array of a meter. As such, the subject invention represents a significant contribution to the art.

The subject invention is shown and described herein in what is considered to be the most practical, and preferred embodiments. It is recognized, however, that departures may be made therefrom, which are within the scope of the invention, and that obvious modifications will occur to one skilled in the art upon reading this disclosure.

The specific devices and methods disclosed are considered to be illustrative and not restrictive. Modifications that come within the meaning and range of equivalents of the disclosed concepts, such as those that would readily occur to one skilled in the relevant art, are intended to be included within the scope of the appended claims.

## Claims

1. A device for determining the concentration of at least one analyte in a physiological sample, said device comprising:
a matrix comprising at least one calibration mark and at least one testing area, wherein said at least one testing area comprises a reagent composition for determining the concentration of an analyte in the physiological sample.

2. The device according to claim 1, wherein said at least one calibration mark is photometrically readable.

3. The device according to claims 1 or 2, wherein said matrix comprises at least two different calibration marks.

4. The device according to claims 1, 2 or 3, wherein said matrix comprises at least two testing areas comprising the same or different reagent composition.

5. The device according to claims 1 to 4, wherein said device is a test strip.

6. A system for determining the concentration of at least one analyte in a physiological sample, said system comprising:
(a) a device according to claims 1 to 5; and
(b) a meter configured to determine the concentration of at least one analyte in the physiological sample applied to said test strip.

7. A method for calibrating a meter using at least one calibration mark positioned on a matrix of a device, said method comprising:
(a) providing a device according to claims 1 to 5,
(b) associating said device with said meter,
(c) illuminating said at least one calibration mark with light;
(d) detecting light from said at least one calibration mark;
(e) calibrating said meter based on said at least one calibration mark.

8. A meter comprising a device according to claims 1 to 5.

9. A kit for calibrating an analyte concentration determination device, said kit comprising:
(a) at least one device according to claims 1 to 5; and
(b) instructions for using said device.

10. The kit according to claim 9, further comprising at least one of:
(a) a meter;
(b) an element for obtaining a physiological sample; and
(c) a control solution.
